# EUROPÄISCHE PATENTSCHRIFT

(11) **EP 1 711 628 B1**
(45) Veröffentlichungstag und Bekanntmachung des Hinweises auf die Patenterteilung: **04.11.2009**
(21) Anmeldenummer: 05701403.7
(22) Anmeldetag: 07.02.2005
(51) Int. Cl.: C12Q 1/68

(54) **VERFAHREN ZUR KALIBRIERUNG UND KONTROLLE VON METHYLIERUNGSANALYSE-METHODEN MIT HILFE VON NICHT-METHYLIERTER DNA**
METHOD FOR THE CALIBRATION AND VERIFICATION OF METHYLATION ANALYSIS METHODS WITH THE AID OF NON-METHYLATED DNA
PROCEDE POUR CALIBRER ET CONTROLER DES METHODES D'ANALYSE DE METHYLATION AU MOYEN D'ADN NON METHYLE

(30) Priorität: 05.02.2004 EP 04090037
(43) Veröffentlichungstag der Anmeldung: 18.10.2006
(73) Patentinhaber: Epigenomics AG, 10178 Berlin (DE)
(72) Erfinder: MODEL, Fabian, 10178 Berlin (DE); SCHUSTER, Matthias, 10178 Berlin (DE); KLUTH, Antje, 10178 Berlin (DE)
(74) Vertreter: Hoppe, Georg Johannes
(86) Internationale Anmeldenummer: PCT/EP2005/001407
(87) Internationale Veröffentlichungsnummer: WO 2005/075671

(56) Entgegenhaltungen:
- WO-A-01/83796
- WO-A-03/085132
- WO-A-2004/051224
- DE-A1- 10 112 515
- US-A1- 2003 082 600
- DEAN F B ET AL: "Comprehensive human genome amplification using multiple displacement amplification" PROCEEDINGS OF THE NATIONAL ACADEMY OF SCIENCES OF USA, NATIONAL ACADEMY OF SCIENCE. WASHINGTON, US, Bd. 99, Nr. 8, 16. April 2002 (2002-04-16), Seiten 5261-5266, XP002297504 ISSN: 0027-8424
- CHEUNG V G AND NELSON S F: "Whole genome amplification using a degenerate oligonucleotide primer allows hundreds of genotypes to be performed on less than one nanogram of genomic DNA" PROCEEDINGS OF THE NATIONAL ACADEMY OF SCIENCES OF USA, NATIONAL ACADEMY OF SCIENCE. WASHINGTON, US, Bd. 93, Dezember 1996 (1996-12), Seiten 14676-14679, XP002126260 ISSN: 0027-8424
- TELENIUS H ET AL: "DEGENERATE OLIGONUCLEOTIDE-PRIMED PCR: GENERAL AMPLIFICATION OF TARGET DNA BY A SINGLE DEGENERATE PRIMER" GENOMICS, ACADEMIC PRESS, SAN DIEGO, US, Bd. 13, 1992, Seiten 718-725, XP000199116 ISSN: 0888-7543
- ZHANG L ET AL: "Whole genome amplification from single cell: Implications for genetic analysis" PROCEEDINGS OF THE NATIONAL ACADEMY OF SCIENCES OF USA, NATIONAL ACADEMY OF SCIENCE. WASHINGTON, US, Bd. 89, Juli 1992 (1992-07), Seiten 5847-5851, XP002130909 ISSN: 0027-8424

## Beschreibung

### Hintergrund der Erfindung

Die vorliegende Erfindung betrifft ein Verfahren zur Methylierungsanalyse gemäß Anspruch 1 und die Verwendung von durch genomweite Amplifizierungsverfahren hergestellter DNA als Standard in der Methylierungsanalyse.

5-Methylcytosin ist die häufigste kovalent modifizierte Base in der DNA eukaryotischer Zellen. Sie spielt eine wichtige biologische Rolle, u.a. bei der Transkriptionsregulation, beim genetischen Imprinting und in der Tumorgenese (zur Übersicht: Millar et al.: Five not four: History and significance of the fifth base. In: The Epigenome, S. Beck and A. Olek (eds.), Wiley-VCH Verlag Weinheim 2003, S. 3-20). Die Identifizierung von 5-Methylcytosin als Bestandteil genetischer Information ist daher von erheblichen Interesse. Ein Nachweis der Methylierung ist allerdings schwierig, da Cytosin und 5-Methylcytosin das gleiche Basenpaarungsverhalten aufweisen. Viele der herkömmlichen, auf Hybridisierung beruhenden Nachweisverfahren vermögen daher nicht zwischen Cytosin und Methylcytosin zu unterscheiden. Zudem geht die Methylierungsinformation bei einer PCR-Amplifikation vollständig verloren.

Die herkömmlichen Methoden zur Methylierungsanalyse arbeiten im wesentlichen nach zwei unterschiedlichen Prinzipien. Zum einen werden methylierungsspezifische Restriktionsenzyme benutzt, zum anderen erfolgt eine selektive chemische Umwandlung von nicht-methylierten Cytosinen in Uracil (sog.: Bisulfit-Behandlung, siehe etwa: DE 101 54 317 A1; DE 100 29 915 A1). Die enzymatisch oder chemisch vorbehandelte DNA wird dann meist amplifiziert und kann auf unterschiedliche Weise analysiert werden (zur Übersicht: WO 02/072880 S. 1 ff). Von großem Interesse sind dabei Verfahren, die in der Lage sind, Methylierung sensitiv und quantitativ zu detektieren. Dies gilt aufgrund der wichtigen Rolle der Cytosin-Methylierung in der Krebsentstehung insbesondere in Hinblick auf diagnostische Anwendungen. Die herkömmlichen Verfahren gewährleisten eine sensitive und quantitative Methylierungsanalyse bisher nur beschränkt.

Zur sensitiven Analyse wird die chemisch vorbehandelte DNA üblicherweise mittels eines PCR-Verfahrens amplifiziert. Über die Verwendung methylierungsspezifischer Primer oder Blocker wird dann eine selektive Amplifikation nur der methylierten (bzw. bei umgekehrten Ansatz: unmethylierten) DNA gewährleistet. Der Einsatz methylierungsspezifischer Primer ist als sog. "methylierungsspezifische PCR" bekannt ("MSP"; Herman et al.: Methylation-specific PCR: a novel PCR assay for methylation status of CpG islands. Proc Natl Acad Sci USA. 1996 Sep 3;93(18):9821-6). Hiermit gelingt vor allem der qualitative Nachweis von methylierter DNA geringer Konzentration. Ein vergleichbar sensitives Verfahren ist die sogenannte "HeavyMethyl"-Methode. Dabei wird eine spezifische Amplifizierung nur der ursprünglich methylierten (bzw. unmethylierten) DNA durch Einsatz von nichtmethylierungsspezifischen Blocker-Oligomeren erreicht (also Blocker-Oligomere, die spezifisch an umgewandelte ehemals unmethylierte Nukleinsäuren hybridisieren; zur Übersicht: WO 02/072880; Cottrell et al.: A real-time PCR assay for DNA-methylation using methylation-specific blockers. Nucl. Acids. Res. 2004 32: e10). Sowohl MSP als auch HeavyMethyl sind als quantifizierbare "Real-Time-Varianten" anwendbar. Diese ermöglichen es, den Methylierungsstatus von Positionen direkt im Verlauf der PCR nachzuweisen, ohne dass eine nachfolgende Analyse der Produkte erforderlich wäre ("MethyLight" - WO00/70090; US 6,331,393).

Eine verläßliche Quantifizierung des Methylierungsstatus über einen linearen Bereich ist mit den oben beschriebenen Verfahren bisher jedoch nur begrenzt möglich. Hierfür ist erforderlich, dass die Assays sowohl mit vollmethylierter wie auch mit nicht-methylierter DNA kalibriert werden (vgl.: Trinh et al.: DNA methylation analysis by MethyLight technology. Methods. 2001 Dec; 25(4): 456-62). Die Herstellung vollmethylierter DNA ist relativ einfach über die Verwendung der SssI-Methylase möglich. Dieses Enzym überführt im Sequenzkontext 5'-CG-3' alle nicht-methylierten Cytosine in 5-Methylcytosin. Problematisch ist allerdings die Herstellung von vollständig nicht-methylierter DNA. Denn ein der SssI-Methylase entsprechendes Enzym, das quantitativ alle Methylgruppen entfernt, steht nicht zur Verfügung. Bisher wird zur Kalibrierung Sperma-DNA verwandt, die über einen geringen Methylierungsgrad verfügt (vgl.: Trinh et al. 2001, a.a.o.). Jedoch ist die Sperma-DNA teilweise methyliert und eignet sich daher als verläßlicher Standard nur bedingt. Auch künstlich hergestellte, kurze unmethylierte Sequenzen wie PCR-Amplifikate sind nur begrenzt einsetzbar, etwa für die Analyse einzelner, definierter Positionen. Für Multiplex-Reaktionen können diese Standards nicht verwendet werden, da die Komplexität der Reaktion dann zu hoch wäre. Zudem erfordert die Entwicklung eines jeden neuen Nachweis-Assays die Herstellung eines neuen definierten Standards. Dagegen würde ein unmethylierter Standard, der die gesamte genomische DNA oder einen repräsentativen Teil hiervon abdeckt, eine zuverlässig quantifizierbare Methylierungsanalyse erlauben. Zudem wäre eine standardisierte und damit einfache, kostengünstige und schnelle Entwicklung neuer Nachweisassays möglich.

In dem Dokument DE 101 12 515 A1 ist die Herstellung und Verwendung eines unmethylierten Standards für die Methylierungsanalyse eines bestimmten Gens beschrieben. Dieser Standard wird jedoch ausgehend von einem definierten genomischen Genfragment synthetisiert und ist somit nur beschränkt in der Methylierungsanalyse einsetzbar.

Weitere Verfahren zur Methylierungsanalyse sind aus den Dokumenten US 2003/082600 A1, WO 03/085132 A und WO 01/83796 A bekannt.

Aufgrund der besonderen biologischen und medizinischen Bedeutung der Cytosin-Methylierung und aufgrund der oben erwähnten Nachteile der zur Zeit verwendeten Standards besteht ein großes technisches Bedürfnis an Methoden, die genomische DNA in vollständig nicht-methylierter Form zur Verfügung stellen. Im folgenden ist ein solches - überraschend einfaches - Verfahren beschrieben. Erfindungsgemäß werden zur Herstellung nicht-methylierter DNA sogenannte genomweite Amplifikationsverfahren verwendet (WGA - whole genome amplification, zur Übersicht: Hawkins et al.: Whole genome amplification--applications and advances. Curr Opin Biotechnol. 2002 Feb; 13(1): 65-7). In diesen Verfahren wird ein großer Teil der genomischen DNA mittels einer DNA-Polymerase und "Random"- oder degenerierter Primer vervielfältigt. Unter "Random" Primern sind solche Primer zu verstehen, die nicht spezifisch an bestimmte Nukleinsäuren, sondern an eine Vielzahl von Nukleinsäuren binden. Dazu gehören Primer die entweder sehr kurz sind (zwischen 5 und 10 bp) oder Primer die als "degenerierte Primer" bezeichnet werden. Unter degenerierten Primern sind solche Primer zu verstehen, die nicht spezifisch an bestimmte Nukleinsäuren binden, weil sie entweder Universalbasen enthalten, die an mehrere verschiedene Nukleotide binden oder aber es wird eine Mischung aus Primern verwendet, die sich jeweils in den "degenerierten" Positionen unterscheiden. Unter Universalbasen versteht man Basen, die an mehrere verschiedene Nukleotide binden (siehe z.B. Katalog Promega : Pyrimidin- oder Purin- spezifische Universalbasen). Beides wird im folgenden als "degenerierter Primer" verstanden. Dabei werden in den Amplifikationen nur nicht-methylierte Cytosintriphosphate angeboten, so dass die Amplifikate vollständig unmethyliert synthetisiert werden. Nach mehreren Amplifikationsrunden tritt die Menge der partiell methylierten Matrizen-DNA im Vergleich zu den neu hergestellten, nicht-methylierten Nukleinsäuren vollständig in den Hintergrund.

Bisher sind unterschiedliche WGA-Verfahren beschrieben. Bei der sog. Primer-Extensions-Präamplifikation (PEP: primer extension preamplification) wird die Amplifikation mittels einer Taq-Polymerase und einer zufälligen Mischung von Oligonukleotidprimern mit einer Länge von etwa 15 Nukleotiden durchgeführt (Zhang et al.: Whole genome amplification from a single cell: implications for genetic analysis. Proc Natl Acad Sci USA. 1992 Jul 1; 89(13): 5847-51). Bei der DOP-PCR (degenerate oligonucleotide primed polymerase chain reaction) wird dagegen nur ein degenerierter Primer eingesetzt (vgl.: Telenius et al.: Degenerate oligonucleotide-primed PCR: general amplification of target DNA by a single degenerate primer. Genomics. 1992 Jul ; 13(3): 718-25). Ein weiteres WGA-Verfahren ist die sog. Linker-Adaptor-PCR. Dabei wird die DNA zunächst mittels eines Restriktionsenzyms verdaut. Anschließend werden an die Restriktionsfragemente Linker ligiert. In der folgenden Amplifikation werden Primer eingesetzt, die spezifisch an die Linker binden (zur Übersicht: Cheung and Nelson: Whole genome amplification using a degenerate oligonucleotide primer allows hundreds of genotypes to be performed on less than one nanogram of genomic DNA. Proc Natl Acad Sci USA. 1996 Dec 10; 93(25): 14676-9 m.w.N.). Die oben beschriebenen, auf PCR-basierenden WGA-Verfahren haben allerdings mehrere Nachteile. So kann es etwa zur Generierung unspezifischer Amplifikationsartefakte kommen. Zudem erfolgt oft nur eine unvollständige Abdeckung aller Genombereiche. Weiterhin entstehen teilweise nur kurze DNA-Fragmente mit einer Länge von weniger als 1 kB. (vgl.: Dean et al.: Comprehensive human genome amplification using multiple displacement amplification. Proc Natl Acad Sci USA. 2002 Apr 16; 99(8): 5261-6 m.w.N.). Das zur Zeit schlagkräftigste Verfahren zur genomweiten Amplifikation ist daher die isotherme "Multiple Displacement Amplification" (MDA, vgl.: Dean et al. 2002 a.a.o.; US Patent 6,124,120). Hierbei wird die genomische DNA mit "Random"-Primern und eine DNA-Polymerase umgesetzt. Es werden dabei Polymerasen eingesetzt, die in der Lage sind, den Nicht-Templat-Strang des DNA-Doppelstrangs während der Amplifikation zu verdrängen (etwa eine ϕ29 Polymerase). Die verdrängten Stränge dienen wiederum als Matrize für die Extension weiterer Primer. Mit diesem Verfahren ist es möglich, aus nur 1-10 Kopien menschlicher genomischer DNA etwa 20-30 µg DNA herzustellen. Dies entspricht einer mehr als 5000-fachen Amplifikation. Die durchschnittliche Produktlänge beträgt dabei mehr als 10 kB, wobei die Amplifikation relativ gleichmäßig über das gesamte Genom erfolgt. Die Reaktion kann direkt aus biologischen Proben erfolgen, etwa aus Blut oder Zellkulturen. Kommerzielle Kits zur MDA sind zur Zeit über zwei Anbieter verfügbar ("GenomiPhi" von Amersham Biosciences, www4.amershambiosciences.com; "Repli-g" von Molecular Staging, www.molecularstaging.com). Auch bereits amplifizierte DNA ist über diese Anbieter erhältlich. Die mittels MDA hergestellte DNA wird in vielfältigen Anwendungen eingesetzt, etwa im Genotyping von Einzelnukleotidpolymorphismen (SNP), im "Chromosomen-Painting", in der Restriktionsfragmentlängenpolymorphismus-Analyse, in der Subklonierung und in der DNA-Sequenzierung. Die MDA ist so insbesondere für genetische, forensische und diagnostische Untersuchungen verwendbar (vgl.: Dean et al. 2002, a.a.o.).

Die Verwendung von durch WGA-Methoden hergestellten DNA als Standard in Verfahren zum Nachweis von 5-Methylcytosin ist bisher noch nicht bekannt. Die im folgenden genauer beschriebenen Anwendungen eröffnen der Methylierungsanalyse daher erstmals Zugang zu genomischer, nicht-methylierter DNA. Aufgrund der besonderen Bedeutung der Cytosinmethylierung und aufgrund der beschriebenen Nachteile des Standes der Technik stellt das Eröffnen dieser vorteilhaften, neuen Technologie einen wichtigen technischen Fortschritt dar.

Ein weiterer Aspekt besteht in der Verwendung von Kalibrierungsstandards, die unmethylierte DNA enthalten, für Verfahren zur Methylierungsanalyse, die auf Verwendung von Mikroarrays basieren, welche dadurch gekennzeichnet sind, dass an ihnen Detektionsoligomere immobilisiert sind. Demnach kann ein auf Mikroarraybenutzung basierendes Verfahren zur Bestimmung des Methylierungsgrades von DNA unter Verwendung von Kalibrierungsstandards verwendet werden, die einerseits unmethylierte DNA und andererseits spezifisch aufmethylierte DNA enthalten. Wobei "spezifisch aufmethylierte" DNA solche DNA bezeichnet, die zu einem bekannten Grad methyliert ist, also eine bekannte Methylierungsrate aufweist. Dieses Verfahren ist dadurch gekennzeichnet, dass anhand der Hybridisierungswerte, die in einem mehrstufigen Prozeß, um ihr Rauschen korrigiert, normalisiert und varianzstabilisert werden, anhand einer berechneten Kalibrierungskurve absolute Werte über die Methylierungsrate erhalten werden können.
Als Quelle solcher DNA kann z.B. Spermien-DNA genommen werden. Da diese aber nicht vollständig unmethyliert ist, ist es bevorzugt hierfür die bereits beschriebene genomische nicht-methylierte DNA zu verwenden. Besonders bevorzugt ist hierbei die Verwendung der nach den beschriebenen Verfahren hergestellten genomischen nicht-methylierten DNA als Kalibrierungsstandard. Für diesen Aspekt ist es jedoch auch denkbar für die Kalibrierung nicht-methylierte DNA aus anderen Quellen zu verwenden, solange sie nachgewiesenermaßen unmethyliert ist(z.B Spermien DNA).

Methoden zur Analyse von Mikroarray-Experimenten, in denen Oligonukleotide zur Detektion von Nukleinsäuren, wie beispielsweise mRNA oder ESTs, oder eben Amplifikate auf einer Oberfläche immobilisiert werden, sind beschrieben. Ein Problem der Analyse von Genexpressions-Mikroarraydaten besteht darin, daß die Variation der Expression unter konstanten Bedingungen von Gen zu Gen nicht konstant ist.(David M. Rocke (2003) Heterogeneity of Variance in Gene Expression Microarray Data. Dieser Artikel ist als Vorabdruck (preprint) erhältlich auf http://www.cipic.ucdavis.edu/∼dmrocke/preprints.html datiert: March 15, 2003).

Ein weiteres Problem ergibt sich jedoch aus der Unvergleichbarkeit von Expressions-Mikroarraydaten aus verschiedenen Mikroarray-Experimenten, weil sich diese nur sehr schwer kalibrieren lassen. Der Ansatz sogenannte "housekeeping" genes als Positivkontrollen zu verwenden, erlaubt zwar eine Aussage darüber, ob eine Hybridisierung tatsächlich erfolgt ist (getestet wird somit nur, ob die Hybridisierungsbedingungen ausreichend waren, um eine Hybridisierung des Analyts zu erlauben), aber eine absolute Quantifizierbarkeit der Expressionsdaten ist damit nicht möglich. Das liegt zum einen daran, daß nicht klar definiert ist, welches Gen unter welchen Bedingungen ein "housekeeping-Gen" darstellt und zum anderen daran, daß es nicht möglich ist, eine bestimmte Menge bekannter DNA zuzugeben, und damit einen absoluten Wert (zum Kalibrieren) zu generieren, weil man dazu im Voraus wissen müßte, wieviele der in der zu untersuchenden Probe vorhandenen Nukleinsäuren tatsächlich an Detektionsoligomer A binden würde, die entsprechende Menge bekannter Zusatz-DNA entspräche dann einer Signalintensität von 100% für genau jenes Detektionsoligomers. In anderen Worten, es ist nicht klar, wieviel Test-DNA bekannter Sequenz (Standard) man einem Experiment zugeben müßte, um einen zum Kalibrieren nötigen Wert (eben entsprechend einem bestimmten definierten Anteil) zu generieren. Daher sind Expressionsstudien immer auf die Angabe relativer Aussagen limitiert. Es läßt sich auch nicht bestimmen, wie groß der Anteil der in der Probe exprimierten mRNA ist, die mit einem bestimmten Oligomer, z.B. Oligo X, hybridisiert, weil die Gesamtheit der Signale nicht der Gesamtheit der enthaltenen mRNAs entsprechen muß.

Bei der hier erwähnten Mikroarray-Methylierungsanalyse (siehe auch Adorjan et al.: Tumour class prediction and discovery by microarray-based DNA methylation analysis. Nucleic Acids Res. 2002 Mar 1; 30(5): e21) sind Oligomere zur Detektion von methylierten (CG-Oligos) und /oder unmethylierten (TG-Oligos) CpG Positionen immobilisiert. Oft werden dedizierte Paare von CG- und TG-Oligos benutzt und ins Verhältnis gesetzt um einen Methylierungsindex zu berechnen. Adorjan et al benutzen log (CG/TG), Gitan et al.(Gitan et al. (2002) Mehylation specific oligonucleotide microarray: a new potential for highthroughput methylation analysis. Genome Res., 12, 158-164) benutzen log(CG)/(log(CG)+log(TG). Somit erhält man Abschätzungen darüber welche CpG Position welcher Probe mehr oder weniger stark methyliert vorliegt. Um statistisch signifikante Marker zu finden reicht diese Methode aus. Eigentlich will man aber echte Methylierungsraten bestimmen, also absolute Werte ermitteln. Dies ist insbesondere wichtig für die anschließende Wahl der Detektionsmethode, für das sogenannte Assay-Format, insbesondere zur Analyse von Proben, die nur wenig DNA beinhalten. Bei den dann zur Anwendung kommenden sogenannten "sensitive detection" Verfahren ist es von Bedeutung ob ein niedriger Wert einer 0-5% Methylierungsrate oder einer 15%-20% Methylierungsrate entspricht.
Bei den genannten Mikroarrays, weiß man, welche Nukleinsäuren (Amplifikate) in der zu hybridisierenden Probe enthalten sind (nur nicht, ob diese ein C oder ein G in der Abfrageposition tragen), und es gibt nur zwei Zustände in denen sie sich unterscheiden können, so daß die Gesamtheit der Signale eines CG- und eines TG-Detektionsoligos konstant ist.
Das in einem Aspekt hierin vorgestellte Verfahren zur Kalibrierung von diesen Mikroarray-Chips hat den Vorteil, erstmalig Zugang zu absoluten Angaben über das Ausmaß der Methylierung bzw. den Grad der Methylierung (level of methylation) zu untersuchender Amplifikate machen zu können.

Sie ist insbesondere geeignet zur Methylierungsanalyse, die dadurch gekennzeichnet ist, daß Mikroarrays verwendet werden, an die pro Abfrageposition im zu detektierenden Amplifikat mindestens zwei Oligonukleotide immobilisiert sind. Außerdem ist sie dadurch gekennzeichnet, daß die gegen die Oligonukleotide zu hybridisierenden Nukleinsäuren (Amplifikate) in ihrer Sequenz an sich bekannt sind, mit Ausnahme von 1 bis drei Abfragepositionen, die aber ihrerseits nur in zwei verschiedenen Varianten auftauchen können. Diese zwei Detektionsoligos unterscheiden sich dadurch, daß sie in der Abfrageposition entweder ein Cytosin oder ein Thymin aufweisen, analog, bei der Analyse des Gegenstranges, entweder ein Guanin oder ein Adenin aufweisen.

Es ist bekannt, daß Hybridisierungsdaten von Mikroarray-Hybridisierungen zunächst hintergrund-korrigiert und dann normalisiert werden, ebenso ist es bekannt, daß diese Daten umgewandelt werden, was als Datentransformation bezeichnet wird, um damit eine Varianzstabilisierung zu erreichen. Varianzstabilisierte Daten sind damit üblichen statistischen Auswertungen zugänglich. Der aktuelle Stand der Forschung auf diesem Gebiet läßt sich zum Beispiel den verschiedenen Arbeiten von Durbin und Rocke oder den anderen hierin aufgeführten Artikeln entnehmen:
- Rocke DM und Lorenzato S (1995), A two-component model for measurement error in analytical chemistry, Technometrics. 37, 176-184;
- Durbin BP et al. (2002), A variance stabilizing transformation for gene-expression microarray data. *Bioinformatics.* **18**(1), 105-110;
- Geller SC et al. (2003), Transformation and Normalization of oligonucleotide microarray data. *Bioinformatics*, **19**(14),1817-1823;
- Durbin BP and Rocke DR (2003), Estimation of transforamtion parameters for microarray data. *Bioinformatics*, **19**(11),1360-1367;
- Durbin BP and Rocke MR (2003), Approximate variance-stabilizing transformations for gene-expression microarray data, *Bioinformatics*, **19**(8), 966-972;
- Durbin BP and Rocke MR (2004), Variance-stabilizing transformations for two-color microarrays, *Bioinformatics*, **20**(5), 660-667.

Ein weiterer Aspekt ist hingegen der Schritt der Kalibrierung der Mikroarraydaten unter Verwendung unmethylierter und damit auch -nun zugänglich gewordener- zu definierten Anteilen -also spezifisch- aufmethylierter DNA, wie im Folgenden näher beschrieben.

### Beschreibung

Erfindungsgemäß wird die durch genomweite Amplifizierungsverfahren hergestellte DNA als Standard in der Methylierungsanalyse verwendet. Erfindungsgemäß ist weiterhin ein Verfahren zur Methylierungsanalyse, das dadurch gekennzeichnet ist, dass
a) eine genomweite Amplifikation durchgeführt wird,
b) die daraus hervorgegangenen Amplifikate in der Methylierungsanalyse als Standard verwendet werden.

Prinzipiell können erfindungsgemäß alle oben beschriebenen WGA-Verfahren eingesetzt werden. Die Reaktionsbedingungen der PEP, DOP-PCR und Linker-PCR gehören zum Stand der Technik (s.o.). Aufgrund der oben beschriebenen Nachteile der auf PCR basierenden WGA-Verfahren wird erfindungsgemäß bevorzugt eine MDA durchgeführt. Auch die Reaktionsbedingungen für ein MDA-Verfahren sind hinreichend bekannt (vgl.: Dean et al 2002, a.a.o.; US-Patente 6,124,120; 6,280,949; 6,642,034; US-Anmeldung 20030143536; Produktinformationen zu den oben erwähnten Genomiphi und Repli-g-Kits). Auch andere Variationen der WGA, insbesondere des MDA-Verfahrens, können erfindungsgemäß zur Herstellung nicht-methylierter DNA verwendet werden. So ist es etwa möglich, die DNA zunächst zu fragmentieren und an die Fragmente Linker zu ligieren. Anschließend werden die Fragmente in Concatamere überführt, die dann über eine MDA amplifiziert werden (multiple strand displacement amplification of concatenated DNA - MDA-CA ; vgl.: US 6,124,120).

Erfindungsgemäß bevorzugt wird jedoch eine herkömmliche MDA benutzt. Vorzugsweise werden zwei Sets von Primern verwendet. Jeweils ein Primerset ist komplementär zu einen Strang der zu amplifizierenden DNA. Bei den Primersets kann es sich um Random-Primer oder degenerierte Primer handeln. Einzelheiten zur Anzahl, Länge und zur Struktur der Primer sind vielfach beschrieben (vgl.: US 6,124,120). So ist etwa bekannt, dass Primer verwendet werden können, die am 5'-Ende nicht komplementär zu der Zielsequenz sind. Hiermit wird die Verdrängung der Primer durch die Polymerase erleichtert. Die 5'-Region der Primer kann zudem funktionale Sequenzen, etwa für einen Promotor tragen (vgl.: US 6,124,120). Der optimale Aufbau der Primer hängt von der Art der verwendeten Polymerase ab, insbesondere von ihrer Prozessivität (vgl.: US 6,124,120). Besonders bevorzugt werden Hexamer-Primer benutzt. Verschiedene Polymerasen sind in der MDA-Reaktion einsetzbar. Die Enzyme müssen entweder alleine oder in Kombination mit Hilfsfaktoren (etwa Helikasen) in der Lage sein, während der Replikation den Nicht-Matrizenstrand der zu replizierenden DNA-Doppelhelix zu verdrängen. Dabei werden bevorzugt Polymerasen eingesetzt, die über keine 5'-3'-Exonuklease-Aktivität verfügen. Alternativ können allerdings auch Primer eingesetzt werden, die am 5'Ende blockiert und daher von den Polymerasen nicht degradierbar sind. Als Polymerase wird besonders bevorzugt die ϕ29-Polymerase verwendet. Diese verfügt über eine sehr hohe Prozessivität, die es ihr erlaubt, sehr effektiv DNA zu synthetisieren, auch wenn extreme Basenzusammensetzungen, kurze Tandemwiederholungen (short tandem repeats) oder Sekundärstrukturen in der DNA auftreten. In dem US-Patent 6,124,120 und in der US-Patenanmeldung 2003/0143536 A1 sind weitere einsetzbare Polymerasen aufgeführt, etwa Bst, Bca oder Phage M2-DNA-Polymerase. Die für die Amplifikation erforderlichen Reaktionsbedingungen sind von der Auswahl der Polymerasen und der Primer abhängig und ergeben sich ebenfalls aus den oben genannten Referenzen. Es ist u.a. auch bekannt, dass über unterschiedliche Methoden eine Detektion und Quantifizierung der amplifizierten DNA erreicht werden kann, etwa über den Einbau markierter Nukleotide, über Verwendung besonderer Detektionssonden oder über Festphasendetektoren (Vgl.: 6,124,120).

In einer bevorzugten Ausführungsform der Erfindung werden die kommerziell erhältlichen Kits zur Synthese der nicht-methylierten DNA verwendet. Besonders bevorzugt werden die Kits "GenomiPhi" (Amersham Biosciences) oder "Repli-g" (Molecular Staging) verwendet. Die Amplifikation erfolgt dabei nach Herstellerangaben. Im wesentlichen wird dabei die zu amplifizierende DNA mit einem Probenpuffer und Random Hexamer Primern versetzt. Die Mischung wird hitzedenaturiert und anschließend gekühlt, so dass es zu einer Bindung der Primer an die DNA kommen kann. Danach werden die verbleibenden Reaktionskomponenten, insbesondere die Desoxynukleosidtriphosphate und die ϕ29-Polymerase hinzugefügt. Die Reaktionsmischung wird dann für etwa 30 Stunden bei 30°C inkubiert. Als Ausgangsmaterial kann etwa DNA verwandt werden, die über die kommerziell verfügbaren Aufreinigungsmethoden isoliert wurde. Für zelluläre Proben wie Blutproben oder Primärzellen aus klinischen Proben kann auch eine alkalische Lyse mit nachfolgender Neutralisation ausreichend sein (vgl.: Produktinformation von Amersham zum GenomiPhi-DNA-Amplifikationskit).

In einer besonders bevorzugten Ausführungsform der Erfindung wird als Standard kommerziell erhältliche, über MDA hergestellte DNA verwendet (s.o.). Dies hat den Vorteil, dass die DNA aufgrund der standardisierten Herstellungsprozesse von gleichbleibender Konzentration und Qualität ist.

Die über die oben beschriebenen Verfahren hergestellte oder käuflich erworbene DNA kann in einer Vielzahl von Methylierungsanalyseverfahren als Standard verwendet werden. Hierzu gehören sowohl Verfahren, die auf dem Einsatz von Restriktionsenzymen beruhen, wie auch Verfahren, die auf einer Bisulfitbehandlung der DNA basieren (vgl.: Fraga and Esteller: DNA Methylation: A Profile of Methods and Applications. Biotechniques 33:632-649, September 2002). Bevorzugt wird zunächst eine Bisulfitumwandlung durchgeführt. Die Bisulfitumwandlung ist dem Fachmann in unterschiedlichen Variationen bekannt (siehe etwa: Frommer et al.: A genomic sequencing protocol that yields a positive display of 5-methylcytosine residues in individual DNA strands. Proc Natl Acad Sci U S A. 1992 Mar 1;89(5):1827-31; Olek, A modified and improved method for bisulphite based cytosine methylation analysis. Nucleic Acids Res. 1996 Dec 15;24(24):5064-6.; DE 100 29 915; DE 100 29 915). Besonders bevorzugt erfolgt die Bisulfitumwandlung in Gegenwart von denaturierenden Lösemitteln, etwa Dioxan, und eines Radikalfängers (vgl.: DE 100 29 915). In einer anderen bevorzugten Ausführungsform wird die DNA nicht chemisch, sondern enzymatisch umgewandelt. Dies ist etwa durch Einsatz von Cytidin-Deaminasen denkbar, die unmethylierte Cytidine schneller umsetzen als methylierte Cytidine. Ein entsprechendes Enzym ist kürzlich identifiziert worden (Bransteitter et al.: Activation-induced cytidine deaminase deaminates deoxycytidine on single-stranded DNA but requires the action of RNase. Proc Natl Acad Sci USA. 2003 Apr 1;100(7):4102-7).

Die umgewandelte DNA kann anhand gängiger molekularbiologischer Verfahren analysiert werden, etwa über Hybidisierung oder Sequenzierung. In einer bevorzugten Variante wird die umgewandelte DNA zunächst amplifiziert. Hierzu sind dem Fachmann unterschiedliche Verfahren bekannt, etwa Ligasekettenreaktionen. Vorzugsweise wird die DNA allerdings über eine Polymerasereaktion amplifiziert. Hierzu sind verschiedene Ausgestaltungen denkbar, etwa die Verwendung isothermer Amplifikationsverfahren. Besonders bevorzugt sind allerdings Polymerasekettenreaktionen (PCR). In einer ganz besonders bevorzugten Ausführungsform erfolgt die PCR unter Verwendung von Primern, die spezifisch nur an Positionen der umgewandelten Sequenz binden, die vorher entweder methyliert oder (bei umgekehrtem Ansatz) unmethyliert) waren (MSP, s.o.). In einer anderen ganz besonders bevorzugten Ausführungsform wird die umgewandelte DNA mit Hilfe von methylierungs- bzw. un-methylierungs-spezifischen Blockern analysiert ("HeavyMethyl"-Methode, s.o.). Die Detektion der PCR-Amplifikate kann über herkömmliche Verfahren erfolgen, etwa über Methoden der Längenmessung wie Gelelektrophorese, Kapillargelelektrophorese und Chromatographie (z.B. HPLC). Auch Massenspektrometrie und Methoden zur Sequenzierung wie die Sanger-Methode, die Maxam-Gilbert-Methode und Sequencing by Hybridisation (SBH) können verwendet werden. In einer bevorzugten Ausführungsform werden die Amplifikate durch Primer-Extension-Verfahren nachgewiesen oder durch methylierungsspezifische Ligationsverfahren (siehe etwa: Gonzalgo & Jones : Rapid quantitation of methylation differences at specific sites using methylation-sensitive single nucleotide primer extension (Ms-SNuPE). Nucleic Acids Res. 1997 Jun 15;25(12):2529-31; DE 100 10 282; DE 100 10 280). In einer anderen bevorzugten Ausführungsform werden die Amplifikate mittels Hybridisierung an Oligomer-Mikroarrays analysiert (vgl.: Adorjan et al.: Tumour class prediction and discovery by microarray-based DNA methylation analysis. Nucleic Acids Res. 2002 Mar 1; 30(5): e21). In einer weiteren besonders bevorzugten Ausführungsform werden die Amplifikate unter Verwendung von PCR-Real-Time-Varianten analysiert (vgl.: Heid et al.: Real time quantitative PCR. Genome Res. 1996 Oct;6(10):986-94, US Patent No. 6,331,393 "MethyLight"). Dabei wird die Amplifikation in Gegenwart eines methylierungsspezifischen, fluoreszenzmarkierten Reporteroligonukleotids durchgeführt. Das Reporteroligonukleotid bindet dann bevorzugt an die zu untersuchende DNA und zeigt deren Amplifikation durch Zunahme oder Abnahme der Fluoreszenz an. Dabei ist es besonders vorteilhaft, wenn die Fluoreszenzveränderung direkt zur Analyse benutzt wird und aus dem Fluorenzenzsignal auf einen Methylierungszustand geschlossen wird. Eine besonders bevorzugte Variante ist dabei das "Taqman"-Verfahren. In einer anderen besonders bevorzugten Ausführungsform wird ein zusätzliches fluoreszenzmarkiertes Oligomer verwendet, das in unmittelbarer Nähe zu dem ersten Reporteroligonukleotid hybridisiert und sich diese Hybridisierung mittels Fluoreszenz-Resonanz-Energietransfer nachweisen läßt ("Lightcycler"-Verfahren).

Eine bevorzugte Ausführungsform der Erfindung ist es, mehrere Fragmente gleichzeitig mittels einer Multiplex-PCR zu amplifizieren. Bei deren Design muss darauf geachtet werden, daß nicht nur die Primer, sondern auch die weiteren eingesetzten Oligonukleotide nicht zueinander komplementär sein dürfen, so daß eine hochgradige Multiplexierung in diesem Fall schwieriger ist als üblich. Erschwerend ist hierbei auch, daß die Bisulfit-bedingte Konversion der Nukleinsäuren deren Komplexität herabsetzt. Jedoch hat man bei der chemisch vorbehandelten DNA den Vorteil, daß aufgrund des unterschiedlichen G- und C-Gehaltes der beiden DNA-Stränge ein Forward-Primer niemals auch als Reverse-Primer fungieren kann, was die Multiplexierung wiederum erleichtert und den oben beschriebenen Nachteil im wesentlichen ausgleicht. Die Detektion der Amplifikate ist wiederum über unterschiedliche Verfahren möglich. Denkbar ist dabei etwa die Verwendung von Real-Time-Varianten. Für Amplifikationen von mehr als vier Genen empfiehlt es sich aber, die Amplifikate auf andere Weise zu detektieren. Bevorzugt ist dabei eine Analyse über Mikroarrays (s.o.).

Eine aktuelle Übersicht über weitere mögliche Methoden zur Methylierungsanalyse findet sich in: Fraga and Esteller 2002,a.a.o.).

In den unterschiedlichen Methoden zur Methylierungsanalyse kann die MDA-DNA auf unterschiedliche Weise als Standard eingesetzt werden. Unter Standard ist dabei zum einen jegliche Art der Negativkontrolle oder Positivkontrolle im Falle des Nachweises von nicht-methylierter DNA zu verstehen. Dies ist insbesondere bei Technologien der Fall, die kleinste Mengen methylierter DNA in einem großen Hintergrund von nicht-methylierter DNA nachweisen und umgekehrt. Dieser Fall wird auch als sogenannte "sensitive detection" bezeichnet. Hier dient die unmethylierte MDA-DNA während der Assay-Entwicklung als Kontrolle der Spezifität des Assays für methylierte DNA und in der Anwendung des Assays als Negativkontrolle. Dabei kann ein Gemisch aus nicht-methylierter DNA und methylierter DNA verwendet werden. Besonders bevorzugt ist es, unterschiedliche Gemische (also bestehend aus unterschiedlichen Anteilen) aus nicht-methylierter und methylierter DNA zu verwenden. Hiermit können dann Kalibrierungskurven erstellt werden. Um diese Gemische herzustellen, verwendet man als Basis bevorzugt die durch MDA hergestellte unmethylierte DNA. Diese Gesamtmenge der Kontroll-DNA wird geteilt und ein Teil davon mittels einer Sssl-Methylase methyliert (s.o.). Dabei wird der andere Teil der nicht-methylierten DNA ebenfalls mit allen Reaktionskomponenten des Methylierungsansatzes bis auf die Methylase umgesetzt. So ist sichergestellt, dass die DNA-Konzentration in beiden Ansätzen identisch ist, und in beiden Ansätzen die gleichen Reaktionskomponenten anwesend sind. Anschließend werden nicht-methylierte und methylierte DNA in unterschiedlichen Verhältnissen gemischt, etwa im Verhältnis 4:0 für 0%, 3:1 für 25%, 2:2 für 50%, 1:3 für 75%, 0:4 für 100%. Für die Entwicklung von Assays für die sensitive Detektion kann es bevorzugt sein, Mischungen mit sehr geringen Konzentrationen an methylierter DNA (etwa 1: 2000-1:10000) herzustellen.

Indem der Quotient der Signale, die für den methylierten Zustand detektiert werden, und der Signale, die für den unmethylierten Zustand detektiert werden, gebildet wird, erhält man die gemessene Methylierungsrate. Trägt man diese gegen die theoretischen Methylierungraten (entsprechend dem Anteil methylierter DNA in den definierten Mischungen) auf und ermittelt die Regression, die durch die Messpunkte geht, erhält man eine Kalibrierungskurve. Anhand dieser Kalibrierungkurve lässt sich über die gemessene Methylierungsrate der Methylierungsgrad der unbekannten Proben bestimmen.
Für Assays die Methylierung über Hybridisierung einer fluoreszierenden Meßsonde quantifizieren (Mikroarrays, MethyLight, andere Hybridisierungsassays in Lösung) ist das gemessene Fluoreszenszsignal über einen weiten Bereich linear von der Konzentration methylierter DNA (wenn die Sonde spezifisch an -vor der Umwandlung- methylierte Nukleinsäuren hybridisiert) abhängig, (JG Wetmur, Hybridization and renaturation kinetics of nucleic acids, Annual Reviews, 1976). Diese Assays können daher kalibriert werden, indem lediglich die unspezifische Hintergrundhybridisierung und der dynamische Bereich der Meßsonden bestimmt wird. Dies kann mit Hilfe von künstlich hergestellter unmethylierter und methylierter DNA geschehen. Die eigentliche Methylierungsrate kann dann durch einfache lineare Interpolation zwischen der Fluoreszenzintensität der unmethylierten DNA (0% Methylierung) und der Fluoreszenzintensitaet der methylierten DNA (100% Methylierung) bestimmt werden. Es ist besonders bevorzugt, dass diese Bestimmung der Methylierungsrate einer gegebenen Probe (sample) aus mehreren wiederholten Messungen erfolgt und die statistische Verteilung des Messrauschens berücksichtigt wird (DM Rocke and S Lorenzato, A two-component model for measurement error in analytical chemistry, Technometrics, 1995, 37, 176-184). Dies wird bevorzugt mit Hilfe klassischer statistischer Verfahren wie der "Maximum Likelihood" oder "Varianzstabilisierung" realisiert.

Im Folgenden wir dieses besonders bevorzugte Verfahren zur Umwandlung von Signalintensitäten aus Methylierungs-Mikroarray-Hybridisierungsexperimenten beschrieben. Die Durchführung solcher methylierungsspezifischen Mikroarray-Hybridisierungsexperimente wurde bereits anderweitig ausführlich beschrieben zum Beispiel von Adorjan et al. (Nucleic Acids Res. 2002 Mar 1;30 (5):e 21). Die Umwandlung der Signalintensitäten in Methylierungswerte jedoch, erfolgt bei Adorjan et al. nach einem anderen Verfahren. Das neue Verfahren ist charakterisiert durch die Verwendung von erfindungsgemäß unmethylierter DNA in einem wesentlichen Schritt, nämlich der Kalibrierung der zuvor vom Hintergrund-Rauschen korrigierten, normalisierten und varianzstabilisierten Hybridisierungsdaten, wie sie insbesondere bei der Methylierungsanalyse mit sogenannten CG- und TG-Oligos vorkommen. Erst die durch die Verwendung unmethylierter DNA ermöglichte Kalibrierung erlaubt letztlich die akkurate Zuordnung von Methylierungs-Signalen, die sich aus Signalintensitäten berechnen lassen, in tatsächliche Methylierungsraten.
Es ist also ein weiterer Aspekt, ein Verfahren bereitzustellen, dass dadurch gekennzeichnet ist, dass es Kalibrierungsstandards zur Auswertung der Hybridisierungsdaten aus Mikroarray-Versuchen benutzt, wobei ein Standard keine methylierte DNA enthält, und der andere Standard DNA eines definierten Grades an Methylierung (z.B. 100%) enthält, um dadurch tatsächliche Methylierungsraten zu bestimmen. Der methylierte Standard wird bevorzugt wie oben beschrieben durch Aufmethylierung mit der Sss1-Methylase hergestellt. Zur Verwendung als Standard, der keine methylierte DNA enthält, wird bevorzugt unmethylierte DNA verwendet. Besonders bevorzugt ist hierbei die Verwendung von nach dem oben beschriebenen Verfahren hergestellter genomischer unmethylierter DNA.
Bevorzugt, aber nicht unbedingt notwendig für die Erstellung einer Kalibrierungskurve, ist die Verwendung mehrerer zu unterschiedlichen Graden methylierter DNA.
Besonders bevorzugt ist jedoch ein Verfahren dass es erlaubt eine akkurate Zuordnung der absoluten Werte anhand einer Kalibrierungskurve zu ermitteln, die unter Zuhilfenahme nur zweier Kalibrierungsstandards (besonders bevorzugt sind hierbei 0% und 100%) berechnet wurde.

Hierbei bezeichnet die Methylierungsrate einen Wert von 0 bis 100, der das Verhältnis der Anteile der tasächlich methylierten DNA und der unmethylierten DNA zueinander in der analysierten Probe (sample) pro Oligonukleotidsonden-Paar (oder pro Abfrageposition (= CpG oder TpG), falls das Oligo nur eine CpG-Site abdeckt) angibt.
Die Kalibrierungsstandards werden benutzt, um eine Kalibrierungskurve zu erstellen, anhand derer -wie im Folgenden näher erläutert- die tatsächlichen Methylierungsraten der untersuchten Proben abgelesen werden können. Das Verfahren der Umwandlung der erhaltenen Hybridisierungswerte in absolute Methylierungsraten wird im Folgenden näher erläutert.
Bevor die Methylierungsdaten eines Methylierungsexperiments (basierend auf Mikroarrays) überhaupt kalibriert werden können, müssen die rohen PIXEL Intensitäts-Angaben der Mikroarray-Laser-Scan-Abbildungen für jedes Oligonukleotidsonden-Paar in Methylierungs-Signalwerte überführt werden. Die rohen Meßdaten einzelner Oligonukleotid-Spotting-Positionen müssen also in Methylierungs-Signale *(methylation signal)* pro Oligonukleotidsonden-Paar (bzw. pro Abfrageposition) der Detektions-Sonden umgewandelt werden. Eine Abfrageposition ist immer jeweils das zu untersuchende Cytosin (oder Thymin) vor einem Guanin (innerhalb eines CpG Dinukleotids). Da Detektions-Oligonukleotidsonden aber auch mehrere solcher CpG-Stellen enthalten können, werden Methylierungssignale im Weiteren pro Oligonukleotidsonden-Paar angegeben und nicht pro Abfrageposition.
Das Auswerteverfahren sei im Folgenden näher erläutert:
Der erste Schritt ist die Korrektur um das Hintergrundrauschen (background correction): Für jeden Spot, also jedes lokalisierte Detektionsoligo, wird der Median der Hintergrundpixel-Intensitäten vom Median der Vordergrundpixel-Intensitäten subtrahiert. Das ergibt eine robuste Abschätzung der Hintergrund-korrigierten Oligonukleotid-Hybridisierungsintensitäten. Dies kann zum Beispiel aber auch über die Verwendung der Formel : X = Imeth- * r² geschehen.

Der zweite Schritt kann als Daten-Normalisierung (normalization of data) bezeichnet werden: Hierzu werden generell klassische Methoden benutzt, die allerdings für die Anwendung auf Methylierungs-Mikroarray-Experimente optimiert werden können, indem von der Annahme ausgegangen wird, daß die Summe der CG-Signale und der TG-Signale innerhalb einer Probe, konstant ist, und zwar über mehrere Mikroarrays hinweg, oder auch nur über mehrere Amplifikate innerhalb eines Mikroarrays hinweg. Dies ist der inhärente Vorteil der Methylierungsanalyse mit CG- und TG-Oligosonden, wie bereits anderweitig beschrieben. Die hintergrund-korrigierten redundanten CG- und TG-Detektionsoligo-Spot-Intensitäten jedes Mikroarrays werden linear skaliert, so daß die Gesamtverteilung der Intensitäten jedes Mikroarrays möglichst identisch sind (einfachster Fall: Median der CG+TG Intensitäten ist identisch für alle Mikroarrays). Die Intensitäten sind bevorzugt redundant, was bedeutet, daß sich auf einem Mikroarray Spots (eine definiert aufgetragene Menge) derselben Sonde mehrfach befinden.
Der dritte Schritt stellt eine Datentransformation dar, der die Varianzstabilisierung zum Ziel hat. Varianzstabilisierung meint hier das Generieren varianzstabilisierter Methylierungssignale im generalisierten logarithmischem Raum. Dies wurde bisher dadurch erreicht, dass, der Logarithmus des Verhältnisses von durch Cytosin (CG) und Thymin (TG) Oligonukleotiden generierten Meßdaten gebildet wurde (wie zuvor von Adorjan et al. Nucleic Acids Res. 2002 Mar 1;30(5):e21. beschrieben). Die generierten Daten sollten varianzstabilisiert sein, damit die Voraussetzungen zur Anwendung etablierter statistischer Auswertemethoden, wie der "maximum likelihood algorithm (ML)" erfüllt sind. Statt der Verwendung des einfachen Logarithmus wird hierfür jedoch bevorzugt das generative Modell von Rocke verwendet, welches das spezifische (intensitätsabhängige, durch die Verwendung von Fluoreszenzintensitäten bedingte) Rauschen, das diesem Meßprozeß inhärent ist, berücksichtigt. Die Verwendung einer generalisierten log Skala anstelle des Logarithmus hat den Vorteil, daß auch negative Intensitätswerte, wie sie bei der Korrektur um das Hintergrundrauschen entstehen können, berücksichtigt werden können und sehr geringe Intensitäten stärker berücksichtigt werden.
Zur näheren Erläuterung der generalisierten log Skala sei hiermit auf die entsprechenden Veröffentlichungen von Rocke in der Fachzeitschrift "Bioinformatics" hingewiesen (Durbin B and Rocke DR (2003) Estimation of transforamtion parameters for microarray data. Bioinformatics vol 19, no 11, pages 1360-1367).
Basierend auf der Verwendung von Negativkontrolloligos, und den damit generierten Intensitätsdaten kann eine globale (also mindestens für alle auf dem Mikroarray immobilisierten Oligonukleotidsondenpaare geltende) Hintergrund-Hybridisierungs-Intensitätsverteilung geschätzt werden. Negativkontrolloligos sind Detektionsoligos, die so entworfen werden, daß sie an keines der zu untersuchenden Amplifikate hybridisieren, die ja bei diesen Hybridisierungsexperimenten zur Methylierungsanalyse im Gegensatz zu Hybridisierungsexperimenten zur Expressionsanalyse bekannt sind. Der Mittelwert (µ, mean) und die Varianz (σ² , variance) für die Normalverteilung (z.B. nach Gauss) werden geschätzt.
Eine zuerst von Durbin und Rocke beschriebene varianzstabilisierende Transformation (Durbin BP et al. (2002). A variance stabilizing transforamtion for gene expression microarray data Bioinformatics, 18(1), 105-110) wird benutzt, um damit CG- und TG-Methylierungs-Meßwerte (=Intensitäten) pro Oligonukleotidsondenpaar in ein Methylierungs-Signal auf einer generalisierten log-Skala zu transformieren. Wie bereits erwähnt, ermöglicht diese generalisierten log Skala eine konsistente Behandlung bzw. Bewertung von niedrigen oder sogar negativen Intensitätssignalen. Diese so erhaltenen Methylierungs-Signale (methylation signals) erfüllen damit nun hinreichend die Voraussetzung zur Anwendung der üblichen Standard-Methoden der Statistik, wie statistischen Tests (zum Beispiel: dem "Student t-test" oder dem "Hotelling multivariate T2 test") und rechtferigen somit deren Verwendung.
Das Methylierungs-Signal hat die Eigenschaft, daß das Hybridisierungsrauschen eine nahezu konstante Varianz über den vollen Bereich aller möglichen Methylierungsniveaus zeigt. Es ist jedoch nur begrenzt aussagekräftig, um Aussagen über absolute Methylierungswerte und daraus abgeleitete Vergleiche zwischen verschiedenen Detektionsoligo-Familien zu machen.
Der vierte Schritt aber, die eigentliche Kalibrierung der nun varianzstabilisierten Daten, hat es zum Ziel, die Methylierungs-Signale (methylation signals) in Methylierungs-Raten (mehylation rates) zu verwandeln.
Die Methylierungs-Signale werden in diesem Schritt der Kalibrierung auf Kontroll-DNA Meßsignale (g log scores) geeicht. Diese werden unter Verwendung unmethylierter Kontroll-DNA und definierter Anteile aufmethylierter Kontroll-DNA in separaten mehrfach wiederholten Mikroarray-Hybridisierungen generiert. Dabei werden die Methylierungs-Signale bestimmt, die den Eichwerten (z.B. 0%, 10%, 90% und 100%) zugeordnet werden. Besonders bevorzugt ist hierbei die Ausführungsform in der nur die Kontroll-DNA-Meßsignale, die den Eichwerten 0% und 100% entsprechen produziert, oder benutzt werden. Mittels "maximum likelihood (ML)" Abschätzung können nun kalibrierte Methylierungs-Raten erhalten werden. Das kalibrierte Methylierungs-Signal ist somit die Methylierungs-Rate und quantifiziert den absoluten Anteil an methylierter DNA im Gemisch aus methylierter und unmethylierter DNA. Während bisher per Mikroarray-Hybridisierung nur bestimmt werden konnte, ob ein Oligonukleotidsondenpaar in verschiedenen Proben signifikant unterschiedlich methyliert war, ist es jetzt möglich, Aussagen beispielsweise darüber zu treffen, ob diese signifikanten Unterschiede von beispielsweise 25% im hohen oder niedrigen Methylierungsniveau liegen, also ob sich der Unterschied aus einer 95%igen Methylierung im einen Fall und einer 70%igen Methlyierung im anderen Fall ergibt, oder aber aus einer 45%igen Methylierung gegenüber einen 20%igen Methylierung.

Ein Verfahren zur Bestimmung von Methylierungsraten von DNA Proben mit Hilfe von Mikroarrays, welche CG- und TG-Oligomere enthalten, ist dadurch gekennzeichnet dass,
die Arrays mit zwei Kalibrierungstandards hybridisiert werden, die definierte Methylierungsraten aufweisen;
anhand der erhaltenen Hybridisierungswerte mit Hilfe einer geeigneten Berechnungsmethode eine Kalibrierungskurve ermittelt wird, und
anhand dieser erstellten Kalibrierungskurve die tatsächlichen Methylierungsraten der untersuchten DNA Proben bestimmt werden.

Bevorzugt ist dieses Verfahren, wobei die zwei Kalibrierungsstandards Methylierungsraten von 0% und 100% aufweisen, bzw, repräsentieren.

Ferner ist ein Verfahren bevorzugt, in welchem mehr als zwei Kalibrierungstandards verwendet werden, die unterschiedliche Methylierungsraten aufweisen.

Besonders bevorzugt ist ein Verfahren, welches dadurch gekennzeichnet ist, dass die tatsächlichen Methylierungsraten in einem mehrstufigen Berechnungsprozeß bestimmt werden, der aus folgenden Schritten besteht:
a) es wird eine Normalisierung der Hybridisierungswerte durchgeführt wird, wodurch Methylierungssignale ermittelt werden,
b) eine Transformation dieser Signale mit dem Ziel einer Varianzstabilisierung wird durchgeführt,
c) die Methylierungsraten werden unter Verwendung der Kalibrierungsstandards und eines geeigneten maximum-likelihood-algorithmus bestimmt.

Besonders bevorzugt ist dieses Verfahren, wenn vor der Normalisierung der Hybridisierungswerte, diese um das der Meßmethode inhärente Hintergrundrauschen korrigiert werden.

Die weiter oben beschriebenen Kontrollen oder Standards lassen sich bei allen Methoden der quantitativen Methylierungsanälyse verwenden: u.a. bei MS-SnuPE, bei Hybridisierung auf Mikroarrays, Hybridisierungsassays in Lösung, direkte Bisulfit-Sequenzierung, bei Real Time PCR (z.B. Heavy Methyl, MSP. vgl. zu den PMR-Werten: Eads et al., CANCER RESEARCH 61, 3410-3418, April 15, 2001),

Eine besonders bevorzugte Verwendung der durch WGA-Verfahren hergestellten DNA und des erfindungsgemäßen Verfahrens liegt in der Diagnose von Krebserkrankungen oder anderen mit einer Veränderung des Methylierungsstatus assoziierten Krankheiten. Hierzu gehören u.a. CNS-Fehlfunktionen, Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion. Das erfindungsgemäße Verfahren eignet sich außerdem zur Vorhersage von unerwünschten Arzneimittelwirkungen und zur Unterscheidung von Zelltypen oder Geweben oder zur Untersuchung der Zelldifferenzierung.

Erfindungsgemäß ist weiterhin ein Kit, der aus bereits über ein WGA-Verfahren amplifizierter DNA sowie aus Reagenzien zur Durchführung einer Bisulfitumwandlung besteht, und optional auch eine Polymerase, Primer und/oder Sonden für eine Amplifikation und Detektion enthält.

### Beispiele:

### Beispiel 1: Verwendung von MDA-DNA für Kalibrierungen

Der Methylierungsgrad einer DNA aus abdominalem Fettgewebe soll mittels Oligonukleotid-Mikroarrays und zum Vergleich mittels des MS-SNuPE-Verfahrens bestimmt werden. Hierzu wird die DNA aus der biologischen Probe mit Hilfe des QIAamp Mini Kits (Qiagen) nach Herstellerangaben extrahiert. Zur Aufnahme einer Kalibrierungskurve werden unterschiedliche Mischungen methylierter und nicht-methylierter DNA hergestellt (0%, 25%, 50%, 75%, 100% methylierter DNA). Die nicht-methylierte DNA wurde über Molecular Staging bezogen, wo sie über eine MDA-Reaktion aus humaner genomischer DNA aus ganzem Blut hergestellt worden war. Bei einer MDA-Reaktion werden alle Methylierungssignale gelöscht (s.o.). Die vollständig methylierte DNA wird aus der MDA-DNA mittels einer Sss1-Methylase (New England Biolabs) hergestellt. Die Synthese erfolgt nach Herstellerangaben. Die übrige nicht-methylierte DNA wird dabei ebenso mit allen Reagenzien bis auf die Sss1-Methylase umgesetzt. So ist sichergestellt, dass die DNA-Konzentration in beiden Ansätzen identisch ist, und in beiden Ansätzen die gleichen Reaktionskomponenten anwesend sind. Anschließend werden nicht-methylierte und methylierte DNA in folgenden Verhältnissen gemischt: 4:0 für 0%, 3:1 für 25%, 2:2 für 50%, 1:3 für 75%, 0:4 für 100%. Die DNA wird anschließend in Gegenwart von Dioxan als denaturiendem Lösemittel Bisulfit-umgewandelt (vgl.: DE 10029 915 A1; deutsche Anmeldung: AZ: 10347396.3). Anschließend werden die erstellten DNA-Gemische und die DNA aus der biologischen Probe (sample) in einer Multiplex-PCR eingesetzt. Dabei werden jeweils 8 Fragmente amplifiziert. Als Primer werden die in Tabelle 1 aufgeführten Oligonukleotide verwendet. Die Amplifikationen werden mittels des QIAGEN HotStarTaq-Kits im wesentlichen nach Herstellerangaben und mit dem folgenden Temperaturprofil durchgeführt: 95°C: 15 min; 45 mal:(95°C: 15sec; 55°C: 30sec; 72°C: 60 sec); 72°C : 10 min. Die Multiplex-PCR-Produkte werden anschließend an ein Oligomer-Mikroarray hybridisiert. Die Sonden-Oligonukleotide sind in Tabelle 2 angegeben. Die Hybridisierung und die Methylierungs-Signal-Ermittlung erfolgen dabei wie bei Adorjan et al., 2002 (a.a.o.) beschrieben. Für jede Probe und jeden Kalibierungsmix werden acht Hybridisierungen durchgeführt. Für die Erstellung von Kalibrierungskurven für eine CpG-Position wird die gemessene Methylierungsrate gegen die theoretische Methylierungsrate aufgetragen. Die gemessene Methylierungsrate ergibt sich aus der Signalintensität einer Oligonukleotidsonde, die für den methylierten Status spezifisch ist, dividiert durch die Gesamtintensität dieser Sonde + einer dazu passenden (also die gleiche CpG-Position abdeckenden) Sonde, die spezifisch ist für den nicht-methylierten Status. Der theoretische Methylierungsstatus entspricht den Methylierungsniveaus der verwendeten definierten Mischungen. Oligonukleotidsonden-Paare, die für Kalibrierungszwecke geeignet sind, weisen monoton steigende Kalibrierungskurven auf. Für die Ms-SNuPE-Reaktion werden die Proben mit den oben aufgeführten Primern in einzelnen PCR-Reaktionen amplifiziert. Die Reaktionsbedingungen sind die gleichen wie für die Multiplex-PCR (s.o.). In der Extensionsreaktion werden als Primer-Bindungsstellen Positionen verwendet, die direkt flankierend zu CpG-Positionen liegen, die denen der Oligonukleotid-Mikroarrays entsprechen. Der Ms-SnuPE-Assay wird entsprechend den Herstellerangaben des MegaBace-SNuPE-Kits durchgeführt. Für die beiden möglichen Varianten des einzubauenden Nucleotides werden mit unterschiedlichen Farbstoffen markierte ddNTPs verwendet. Für jeden SNuPE-Assay werden vier Messungen parallel durchgeführt. Die von der SNP-Profile-Software (Amersham) ermittelten Signalintensitäten (Imeth- für unmethyliert-spezifische Sonden und Imeth+ für methyliert-spezifische Sonden) der beiden verwendeten Farbstoffe werden entsprechend des Quotienten Imeth⁺/(Imeth⁻ + Imeth⁺ verwendet, um die gemessene Methylierungsrate zu ermittlen. Durch das Auftragen dieser Werte gegen die theoretische Methylierungsrate erhält man wiederum eine Kalibrierungskurve, die monoton steigend sein sollte. Die so erzeugten monoton steigenden Kalibrierungskurven werden verwendet, um aus der gemessenen Methylierungsrate von Proben-DNA die tatsächliche Methylierung zu ermitteln.
Die mit den beiden Methoden Mikroarray-Analyse (chip) und SNuPE ermittelten und an entsprechenden Kalibrierungskurven korrigierten Methylierungsraten in Proben-DNA stimmen für die gezeigten CpG-Positionen gut überein. Diese Daten zeigen, dass die über MDAhergestellte nicht-methylierte DNA bzw. entsprechende Gemische mit methylierter DNA sehr gut als Standard in der Methylierungsanalyse verwendet werden kann.

### Beispiel 2:

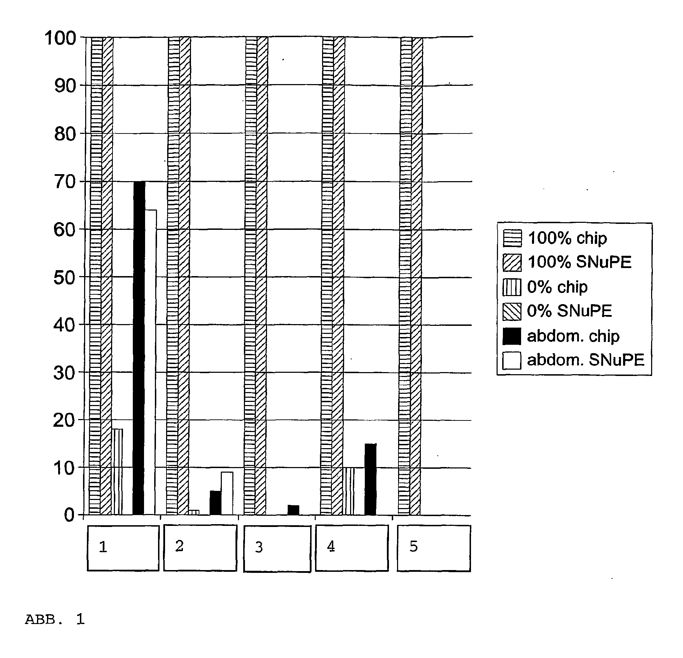

Die Ergebnisse eines analog durchgeführten Experimentes mit jedoch zum Teil differierenden Oligosequenzen sind in Abbildung 1 dargestellt. Die y-Achse zeigt den Prozentsatz an Methylierung, die x-Achse die Hybridisierung an verschiedene Oligonukleotide bzw. verschiedene SNuPE-Assays.

### Abbildungen

### Abbildung 1:

Die Abbildung 1 zeigt die Ergebnisse eines analog zu Beispiel 1 beschriebenen Experimentes. Die y-Achse zeigt den ermittelten Prozentsatz an Methylierung, die x-Achse gibt an dass verschiedene Amplifikate (1-5) untersucht wurden. Die verschiedenen Schraffuren zeigen an mit welcher Methode dieser Wert bestimmt worden ist. Die Hybridisierung an verschiedene immobilisierte Oligonukleotide wird hierin als "chip" indiziert und die alternativ eingesetzte Methode ist der SNuPE-Assay, bezeichnet als "SnuPE". Weiterhin ist angegeben, ob es sich um 100% aufmethylierte DNA (100%), 0% methylierte also unmethylierte DNA (0%) oder natürlicherweise vorkommende DNA aus dem menschlichen Abdominalgewebe handelt. Die mit den beiden Methoden ermittelten und an entsprechenden Kalibrierungskurven korrigierten Methylierungsraten in Proben-DNA stimmen für die gezeigten CpG-Positionen gut überein.

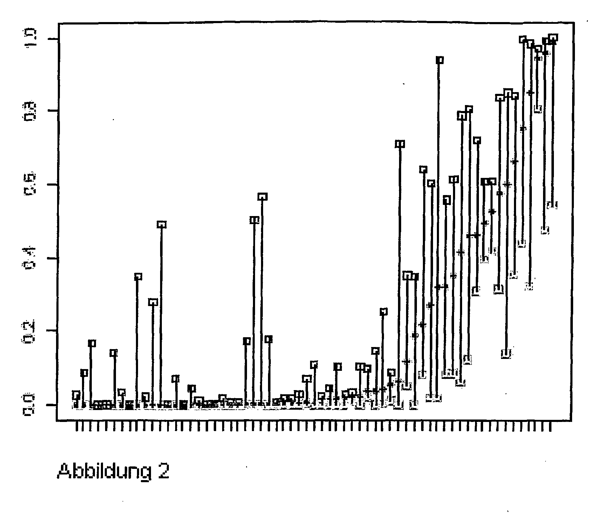

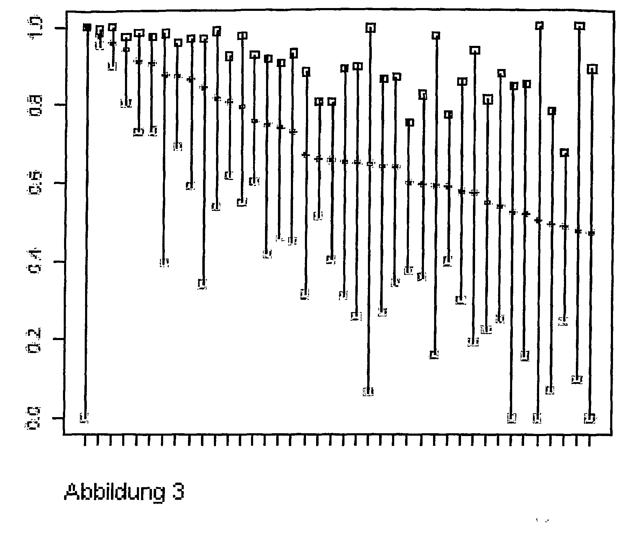

Die Abbildungen 2 und 3 zeigen Plots, die an der Y-Achse die absoluten Methylierungsraten (methylation levels) in Brustkrebsproben und Lymphozyten Proben angeben. Für jedes Detektionsoligo sind Minimum- und Maximum-Hybridisierungsintensitäten bestimmt worden. Dies gelang durch Hybridisierung mit völlig unmethylierter menschlicher DNA (Molecular Staging, new haven, CT) einerseits und enzymatisch methylierter Kontroll-DNA (Sss1; New England Biomedical), als Kalibrierungsstandards, andererseits. Der Anteil der Methylierung einer Probe an einer bestimmten CpG Position wird ermittelt durch lineare Interpolation zwischen der entsprechenden Minimumintensität (0% Methylierung von CG-Oligos, 100% Methylierung für TG-oligos) und der Maximumintensität (100% Methylierung von CG-Oligos, 0% Methylierung für TG-oligos). Die lineare Interpolation wird durch Anwendung eines "Maximum Likelihood Algorithmus" durchgeführt, der die hybridisierungsspezifische Fehlerverteilung (siehe hierzu Rocke and Durbin, (2001) A model for measurement error for gene expression arrays, Journal of Computational Biology, 8, 557-569) berücksichtigt. Wahrscheinlichkeiten (likelihoods) von CG- und TG-Oligomeren derselben CpG Position werden kombiniert in einen Wert für den Methylierungsanteil. Abbildungen 2 und 3 zeigen die Verteilung von ermittelten Methylierungsraten für jede CpG Position, in der Reihenfolge geordnet nach dem Median der Methylierungsrate.

In Abbildung 2 sind Ergebnisse von Lymphozyten Proben gezeigt.

In Abbildung 3 sind Ergebnisse von Brustkrebsdaten gezeigt.

**Tabelle 1: Primer für die Multiplex-Amplifikation**

| Gen Name | RefSeq-ID | Primer Orientierung | Sequenz |
|---|---|---|---|
| ERS1 | NM_000125 & NM_002920 | forward | AGGAGGGGGAATTAAATAGA |
| | | reverse | ACAATAAAACCATCCCAAATAC |
| AR | NM_000044 | forward | GTAGTAGTAGTAGTAAGAGA |
| | | reverse | ACCCCCTAAATAATTATCCT |
| CDKN2a | NM_000077 | forward | GGGGTTGGTTGGTTATTAGA |
| | | reverse | AACCCTCTACCCACCTAAAT |
| CDKN2B | NM_004936 | forward | GGTTGGTTGAAGGAATAGAAAT |
| | | reverse | CCCACTAAACATACCCTTATTC |
| GSTP1 | NM_000852 | forward | ATTTGGGAAAGAGGGAAAG |
| | | reverse | TAAAAACTCTAAACCCCATCC |
| TP73 | NM_005427 | forward | AGTAAATAGTGGGTGAGTTATGAA |
| | | reverse | GAAAAACCTCTAAAAACTACTCTC C |
| MLH1 | NM_000249 | forward | TAAGGGGAGAGGAGGAGTTT |
| | | reverse | ACCAATTCTCAATCATCTCTTT |
| MGMT | NM_002412 | forward | AAGGTTTTAGGGAAGAGTGTTT |
| | | reverse | ACCTTTTCCTATCACAAAAATAA |

**Tabelle 2: Oligonukleotidsonden**

| Name des Oligonukleotids | Sequenz |
|---|---|
| Oligonukleotidsonden für ERS1 | |
| ERS1:204A209 | ATTTAGTAGCGACGATAAGT |
| ERS1:204A204 | GTAGCGACGATAAGTAAAGT |
| ERS1:204A217 | TTAGTAGCGACGATAAGTAAA |
| ERS1:204A2212 | TTTATTTAGTAGCGACGATAAG |
| ERS1:204B237 | TTAGTAGTGATGATAAGTAAAGT |
| ERS1:204B2413 | TTTTATTTAGTAGTGATGATAAGT |
| ERS1:204B2512 | TTTATTTAGTAGTGATGATAAGTAA |
| ERS1:204B2511 | TTATTTAGTAGTGATGATAAGTAAA |
| ERS1:248A195 | GGGATCGTTTTAAATCGAG |
| ERS1:248A204 | GGATCGTTTTAAATCGAGTT |
| ERS1:248A206 | TGGGATCGTTTTAAATCGAG |
| ERS1:248A213 | GATCGTTTTAAATCGAGTTGT |
| ERS1:248B216 | TGGGATTGTTTTAAATTGAGT |
| ERS1:248B223 | GATTGTTTTAAATTGAGTTGTG |
| ERS1:248B224 | GGATTGTTTTAAATTGAGTTGT |
| ERS1:248B228 | TTTGGGATTGTTTTAAATTGAG |
| ERS1:607A183 | GTTCGCGGTTACGGATTA |
| ERS1:607A193 | GTTCGCGGTTACGGATTAT |
| ERS1:607A194 | TGTTCGCGGTTACGGATTA |
| ERS1:608A203 | GTTCGCGGTTACGGATTATG |
| ERS1:607B213 | GTTTGTGGTTATGGATTATGA |
| ERS1:607B219 | TATTTTGTTTGTGGTTATGGA |
| ERS1:607B215 | TTGTTTGTGGTTATGGATTAT |
| ERS1:607B227 | TTTTGTTTGTGGTTATGGATTA |
| ERS1:451A193 | TATCGGATTCGTAGGTTTT |
| ERS1:451A204 | TTATCGGATTCGTAGGTTTT |
| ERS1:451A206 | TTTTATCGGATTCGTAGGTT |
| ERS1:451A207 | GTTTTATCGGATTCGTAGGT |
| ERS1:451B218 | GGTTTTATTGGATTTGTAGGT |
| ERS1:451B226 | TTTTATTGGATTTGTAGGTTTT |
| ERS1:451B227 | GTTTTATTGGATTTGTAGGTTT |
| ERS1:451B237 | GTTTTATTGGATTTGTAGGTTTT |
| Oligonukleotidsonden für AR | |
| AR:971A188 | AGTATTTTCGGACGAGGA |
| AR:971A183 | TTTCGGACGAGGATGATT |
| AR:971A196 | TATTTTCGGACGAGGATGA |
| AR:971A1910 | TTAGTATTTTCGGACGAGG |
| AR:971B218 | AGTATTTTTGGATGAGGATGA |
| AR:971B2112 | TGTTAGTATTTTTGGATGAGG |
| AR:971B213 | TTTTGGATGAGGATGATTTAG |
| AR:971B217 | GTATTTTTGGATGAGGATGAT |
| AR:1137°164 | GTAGCGGGAGAGCGAG |
| AR:1137°175 | AGTAGCGGGAGAGCGAG |
| AR:1137°186 | TAGTAGCGGGAGAGCGAG |
| AR:1137B183 | TAGTGGGAGAGTGAGGGA |
| AR:1137B185 | AGTAGTGGGAGAGTGAGG |
| AR:1137B197 | GTAGTAGTGGGAGAGTGAG |
| AR:1137B174 | GTAGTGGGAGAGTGAGG |
| AR:869A195 | ATAGTCGTAGTCGGTTTTG |
| AR:869A208 | TTTATAGTCGTAGTCGGTTT |
| AR:869A219 | TTTTATAGTCGTAGTCGGTTT |
| AR:869A2111 | ATTTTTATAGTCGTAGTCGGT |
| AR:869B193 | AGTTGTAGTTGGTTTTGGA |
| AR:869B2212 | AATTTTTATAGTTGTAGTTGGT |
| AR:869B2313 | TAATTTTTATAGTTGTAGTTGGT |
| AR:869B2414 | GTAATTTTTATAGTTGTAGTTGGT |
| AR:814A228 | AAGTTTATCGTAGAGGTTTTAT |
| AR:814A2212 | TTTTAAGTTTATCGTAGAGGTT |
| AR:814A2310 | TTAAGTTTATCGTAGAGGTTTTA |
| AR:814A238 | AAGTTTATCGTAGAGGTTTTATA |
| AR:814B228 | AAGTTTATTGTAGAGGTTTTAT |
| AR:814B2210 | TTAAGTTTATTGTAGAGGTTTT |
| AR:814B2212 | TTTTAAGTTTATTGTAGAGGTT |
| AR:814B2211 | TTTAAGTTTATTGTAGAGGTTT |
| Oligonukleotidsonden für CDKN2A | |
| CDKN2A:2147A173 | GGGCGTTGTTTAACGTA |
| CDKN2A:2147A183 | GGGCGTTGTTTAACGTAT |
| CDKN2A:2147B195 | GGGGGTGTTGTTTAATGTA |
| CDKN2A:2147B194 | GGGGTGTTGTTTAATGTAT |
| CDKN2A:2157A217 | TGTTTAACGTATCGAATAGTT |
| CDKN2A:2157A227 | TGTTTAACGTATCGAATAGTTA |
| CDKN2A:2157A228 | TTGTTTAACGTATCGAATAGTT |
| CDKN2A:2157A238 | TTGTTTAACGTATCGAATAGTTA |
| CDKN2A:2157B229 | GTTGTTTAATGTATTGAATAGT |
| CDKN2A:2157B239 | GTTGTTTAATGTATTGAATAGTT |
| CDKN2A:2157B249 | GTTGTTTAATGTATTGAATAGTTA |
| CDKN2A:2183A176 | GGAGGTCGATTTAGGTG |
| CDKN2A:2183A186 | GGAGGTCGATTTAGGTGG |
| CDKN2A:2183B186 | GGAGGTTGATTTAGGTGG |
| CDKN2A:2172A183 | TTACGGTCGGAGGTCGAT |
| CDKN2A:2172A165 | AGTTACGGTCGGAGGT |
| CDKN2A:2172A176 | TAGTTACGGTCGGAGGT |
| CDKN2A:2172A188 | AATAGTTACGGTCGGAGG |
| CDKN2A:2172B198 | AATAGTTATGGTTGGAGGT |
| CDKN2A:2172B209 | GAATAGTTATGGTTGGAGGT |
| CDKN2A:2172B194 | GTTATGGTTGGAGGTTGAT |
| CDKN2A:2172B203 | TTATGGTTGGAGGTTGATTT |
| Oligonukleotidsonden für CDKN2B | |
| CDKN2B:2279A185 | GTTTACGGTTAACGGTGG |
| CDKN2B:2279A183 | TTACGGTTAACGGTGGAT |
| CDKN2B:2279A197 | AAGTTTACGGTTAACGGTG |
| CDKN2B:2279A209 | TTAAGTTTACGGTTAACGGT |
| CDKN2B:2279B206 | AGTTTATGGTTAATGGTGGA |
| CDKN2B:2279B216 | AGTTTATGGTTAATGGTGGAT |
| CDKN2B:2279B223 | TTATGGTTAATGGTGGATTATT |
| CDKN2B:2279B2210 | GTTAAGTTTATGGTTAATGGTG |
| CDKN2B:2330A156 | GGAATGCGCGAGGAG |
| CDKN2B:2330A165 | GAATGCGCGAGGAGAA |
| CDKN2B:2330A175 | GAATGCGCGAGGAGAAT |
| CDKN2B:2330A184 | AATGCGCGAGGAGAATAA |
| CDKN2B:2330B186 | GGAATGTGTGAGGAGAAT |
| CDKN2B:2330B196 | GGAATGTGTGAGGAGAATA |
| CDKN2B:2330B205 | GAATGTGTGAGGAGAATAAG |
| CDKN2B:2329B206 | GGAATGTGTGAGGAGAATAA |
| CDKN2B:2234A168 | AGAGAGTGCGTCGGAG |
| CDKN2B:2234A167 | GAGAGTGCGTCGGAGT |
| CDKN2B:2234A166 | AGAGTGCGTCGGAGTA |
| CDKN2B:2234A176 | AGAGTGCGTCGGAGTAG |
| CDKN2B:2234B178 | AGAGAGTGTGTTGGAGT |
| CDKN2B:2234B188 | AGAGAGTGTGTTGGAGTA |
| CDKN2B:2234B187 | GAGAGTGTGTTGGAGTAG |
| CDKN2B:2234B198 | AGAGAGTGTGTTGGAGTAG |
| CDKN2B:2268A193 | TGTCGTTAAGTTTACGGTT |
| CDKN2B:2268A194 | GTGTCGTTAAGTTTACGGT |
| CDKN2B:2268A205 | AGTGTCGTTAAGTTTACGGT |
| CDKN2B:2268A203 | TGTCGTTAAGTTTACGGTTA |
| CDKN2B:2268B215 | AGTGTTGTTAAGTTTATGGTT |
| CDKN2B:2268B216 | GAGTGTTGTTAAGTTTATGGT |
| CDKN2B:2268B224 | GTGTTGTTAAGTTTATGGTTAA |
| CDKN2B:2268B225 | AGTGTTGTTAAGTTTATGGTTA |
| Oligonukleotidsonden für MGMT | |
| MGMT:597A188 | GGATTATTCGGGTACGTG |
| MGMT:597A184 | TATTCGGGTACGTGGTAG |
| MGMT:597A186 | ATTATTCGGGTACGTGGT |
| MGMT:597A196 | ATTATTCGGGTACGTGGTA |
| MGMT:597B193 | ATTTGGGTATGTGGTAGGT |
| MGMT:597B205 | TTATTTGGGTATGTGGTAGG |
| MGMT:597B204 | TATTTGGGTATGTGGTAGGT |
| MGMT:597B2212 | TTTAGGATTATTTGGGTATGTG |
| MGMT:621A174 | TGTACGTTCGCGGATTA |
| MGMT:621A183 | GTACGTTCGCGGATTATT |
| MGMT:621A185 | TTGTACGTTCGCGGATTA |
| MGMT:621A184 | TGTACGTTCGCGGATTAT |
| MGMT:621B217 | GTTTGTATGTTTGTGGATTAT |
| MGMT:621B224 | TGTATGTTTGTGGATTATTTTT |
| MGMT:621B223 | GTATGTTTGTGGATTATTTTTG |
| MGMT:621B225 | TTGTATGTTTGTGGATTATTTT |
| MGMT:394A197 | TTTTGGACGGTATCGTTTA |
| MGMT:394A206 | TTTGGACGGTATCGTTTATT |
| MGMT:394A208 | TTTTTGGACGGTATCGTTTA |
| MGMT:394A213 | GGACGGTATCGTTTATTATAG |
| MGMT:394B2111 | TAGTTTTTGGATGGTATTGTT |
| MGMT:394B229 | GTTTTTGGATGGTATTGTTTAT |
| MGMT:394B234 | TGGATGGTATTGTTTATTATAGG |
| MGMT:394B237 | TTTTGGATGGTATTGTTTATTAT |
| MGMT:530A173 | TTTCGAGTAGGATCGGG |
| MGMT:530A184 | GTTTCGAGTAGGATCGGG |
| MGMT:530A183 | TTTCGAGTAGGATCGGGA |
| MGMT:530A193 | TTTCGAGTAGGATCGGGAT |
| MGMT:530B194 | GTTTTGAGTAGGATTGGGA |
| MGMT:530B193 | TTTTGAGTAGGATTGGGAT |
| MGMT:530B203 | TTTTGAGTAGGATTGGGATT |
| MGMT:530B204 | GTTTTGAGTAGGATTGGGAT |
| Oligonukleotidsonden für MLH1 | |
| MLH1:1753A176 | GAAGAGCGGATAGCGAT |
| MLH1:1753A185 | AAGAGCGGATAGCGATTT |
| MLH1:1753A184 | AGAGCGGATAGCGATTTT |
| MLH1:1753A193 | GAGCGGATAGCGATTTTTA |
| MLH1:1753B198 | AGGAAGAGTGGATAGTGAT |
| MLH1:1753B2110 | ATAGGAAGAGTGGATAGTGAT |
| MLH1:1753B214 | AGAGTGGATAGTGATTTTTAA |
| MLH1:1753B226 | GAAGAGTGGATAGTGATTTTTA |
| MLH1:2026A186 | AAATGTCGTTCGTGGTAG |
| MLH1:2026A197 | AAAATGTCGTTCGTGGTAG |
| MLH1:2026A1910 | GTTAAAATGTCGTTCGTGG |
| MLH1:2026A209 | TTAAAATGTCGTTCGTGGTA |
| MLH1:2026B195 | AATGTTGTTTGTGGTAGGG |
| MLH1:2026B207 | AAAATGTTGTTTGTGGTAGG |
| MLH1:2026B218 | TAAAATGTTGTTTGTGGTAGG |
| MLH1:2026B2110 | GTTAAAATGTTGTTTGTGGTA |
| MLH1:1770A186 | TTTTAACGCGTAAGCGTA |
| MLH1:1770A194 | TTAACGCGTAAGCGTATAT |
| MLH1:1770A195 | TTTAACGCGTAAGCGTATA |
| MLH1:1770A203 | TAACGCGTAAGCGTATATTT |
| MLH1:1770B239 | GATTTTTAATGTGTAAGTGTATA |
| MLH1:1770B234 | TTAATGTGTAAGTGTATATTTTT |
| MLH1:1770B249 | GATTTTTAATGTGTAAGTGTATAT |
| MLH1:1770B259 | GATTTTTAATGTGTAAGTGTATATT |
| MLH1:1939A173 | GAACGTGAGTACGAGGT |
| MLH1:1939A183 | GAACGTGAGTACGAGGTA |
| MLH1:1939A185 | AAGAACGTGAGTACGAGG |
| MLH1:1939A186 | GAAGAACGTGAGTACGAG |
| MLH1:1939B207 | GGAAGAATGTGAGTATGAGG |
| MLH1:1939B208 | AGGAAGAATGTGAGTATGAG |
| MLH1:1939B216 | GAAGAATGTGAGTATGAGGTA |
| MLH1:1939B213 | GAATGTGAGTATGAGGTATTG |
| Oligonukleotidsonden für TP73 | |
| TP73:750A174 | GATTCGTTGCGGTTAGA |
| TP73:750A184 | GATTCGTTGCGGTTAGAG |
| TP73:750A183 | ATTCGTTGCGGTTAGAGA |
| TP73:750A185 | GGATTCGTTGCGGTTAGA |
| TP73:750B205 | GGATTTGTTGTGGTTAGAGA |
| TP73:750B213 | ATTTGTTGTGGTTAGAGAATT |
| TP73:750B214 | GATTTGTTGTGGTTAGAGAAT |
| TP73:750B223 | ATTTGTTGTGGTTAGAGAATTT |
| TP73:1082A164 | GGTGCGCGTAGAGAAT |
| TP73:1082A166 | TTGGTGCGCGTAGAGA |
| TP73:1082A165 | TGGTGCGCGTAGAGAA |
| TP73:1082A174 | GGTGCGCGTAGAGAATA |
| TP73:1082B1810 | AGGTTTGGTGTGTGTAGA |
| TP73:1082B195 | TGGTGTGTGTAGAGAATAA |
| TP73:1082B207 | TTTGGTGTGTGTAGAGAATA |
| TP73:1082B217 | TTTGGTGTGTGTAGAGAATAA |
| TP73:858A186 | GGATATCGGTTCGGAGTT |
| TP73:858A189 | AGAGGATATCGGTTCGGA |
| TP73:858A195 | GATATCGGTTCGGAGTTAG |
| TP73:858A193 | TATCGGTTCGGAGTTAGAT |
| TP73:858B2011 | GTAGAGGATATTGGTTTGGA |
| TP73:858B208 | GAGGATATTGGTTTGGAGTT |
| TP73:858B224 | ATATTGGTTTGGAGTTAGATTA |
| TP73:858B235 | GATATTGGTTTGGAGTTAGATTA |
| TP73:1135A204 | ATATCGAACGGGATTTAGAG |
| TP73:1135A2112 | TTTTTTAAATATCGAACGGGA |
| TP73:1135A228 | TTAAATATCGAACGGGATTTAG |
| TP73:1135A229 | TTTAAATATCGAACGGGATTTA |
| TP73:1135B224 | ATATTGAATGGGATTTAGAGTT |
| TP73:1135B237 | TAAATATTGAATGGGATTTAGAG |
| TP73:1135B248 | TTAAATATTGAATGGGATTTAGAG |
| TP73:1135B2413 | TTTTTTTAAATATTGAATGGGATT |
| Oligonukleotidsonden für GSTP1 | |
| GSTP1:1900A157 | GGGAGTTCGCGGGAT |
| GSTP1:1900A166 | GGAGTTCGCGGGATTT |
| GSTP1:1900A175 | GAGTTCGCGGGATTTTT |
| GSTP1:1900A185 | GAGTTCGCGGGATTTTTT |
| GSTP1:1900B177 | GGGAGTTTGTGGGATTT |
| GSTP1:1900B187 | GGGAGTTTGTGGGATTTT |
| GSTP1:1900B196 | GGAGTTTGTGGGATTTTTT |
| GSTP1:1900B206 | GGAGTTTGTGGGATTTTTTA |
| GSTP1:2007A196 | GAGTTTCGTCGTCGTAGTT |
| GSTP1:2007B198 | TGGAGTTTTGTTGTTGTAG |
| GSTP1:2007B219 | TTGGAGTTTTGTTGTTGTAGT |
| GSTP1:2126A207 | GGTTTTTCGTTTATTTCGAG |
| GSTP1:2126A216 | GTTTTTCGTTTATTTCGAGAT |
| GSTP1:2126A218 | AGGTTTTTCGTTTATTTCGAG |
| GSTP1:2126A226 | GTTTTTCGTTTATTTCGAGATT |
| GSTP1:2126B217 | GGTTTTTTGTTTATTTTGAGA |
| GSTP1:2126B227 | GGTTTTTTGTTTATTTTGAGAT |
| GSTP1:2126B228 | AGGTTTTTTGTTTATTTTGAGA |
| GSTP1:2126B2310 | GTAGGTTTTTTGTTTATTTTGAG |
| GSTP1:2142A153 | ATTCGGGACGGGGGT |
| GSTP1:2142A154 | GATTCGGGACGGGGG |
| GSTP1:2142A155 | AGATTCGGGACGGGG |
| GSTP1:2142A156 | GAGATTCGGGACGGG |
| GSTP1:2142B174 | GATTTGGGATGGGGGTT |
| GSTP1:2142B175 | AGATTTGGGATGGGGGT |
| GSTP1:2142B176 | GAGATTTGGGATGGGGG |
| GSTP1:2142B184 | GATTTGGGATGGGGGTTT |
| Oligonukleotidsonden für CDH13 C | |
| CDH13:137A1810 | ATGTTATTTTCGCGGGGT |
| CDH13:137B1910 | ATGTTATTTTTGTGGGGTT |
| CDH13:137B2011 | GATGTTATTTTTGTGGGGTT |
| CDH13:153A174 | TTTTCGCGAGGTGTTTA |
| CDH13:153A184 | TTTTCGCGAGGTGTTTAT |
| CDH13:153A185 | TTTTTCGCGAGGTGTTTA |
| CDH13:153A195 | TTTTTCGCGAGGTGTTTAT |
| CDH13:153B206 | GTTTTTTGTGAGGTGTTTAT |
| CDH13:153B215 | TTTTTTGTGAGGTGTTTATTT |
| CDH13:153B216 | GTTTTTTGTGAGGTGTTTATT |
| CDH13:153B226 | GTTTTTTGTGAGGTGTTTATTT |
| CDH13:187A173 | AAACGAGGGAGCGTTAG |
| CDH13:187A174 | AAAACGAGGGAGCGTTA |
| CDH13:187A175 | TAAAACGAGGGAGCGTT |
| CDH13:187A185 | TAAAACGAGGGAGCGTTA |
| CDH13:187B183 | AAATGAGGGAGTGTTAGG |
| CDH13:187B193 | AAATGAGGGAGTGTTAGGA |
| CDH13:187B194 | AAAATGAGGGAGTGTTAGG |
| CDH13:187B197 | TGTAAAATGAGGGAGTGTT |
| CDH13:22°203 | GGTCGTTAGTTTTTCGTGTA |
| CDH13:22B203 | GGTTGTTAGTTTTTTGTGTA |
| CDH13:22B213 | GGTTGTTAGTTTTTTGTGTAA |
| CDH13:22B214 | TGGTTGTTAGTTTTTTGTGTA |
| CDH13:22B223 | GGTTGTTAGTTTTTTGTGTAAT |

Alle in dieser Beschreibung angegebenen Literaturstellen und Referenzen werden hiermit in ihrem vollen Umfang in den Offenbarungsgehalt der vorliegenden Anmeldung eingeschlossen

## Patentansprüche

1. Verfahren zur Methylierungsanalyse, **dadurch gekennzeichnet, dass**
a) eine genomweite Amplifikation durchgeführt wird,
b) die in a) generierten Amplifikate als Standard in der Methylierungsanalyse verwendet werden.

2. Verwendung von durch genomweite Amplifizierungsverfahren hergestellter DNA als Standard in der Methylierungsanalyse.

3. Verfahren oder Verwendung nach Anspruch 1 oder 2, **dadurch gekennzeichnet, dass** als Amplifikationsverfahren entweder
a) PEP,
b) DOP-PCR,
c) Linker-PCR oder
d) Multiple Displacement Amplifikation (MDA) durchgeführt wird.

4. Verfahren oder Verwendung nach Anspruch 3, **dadurch gekennzeichnet, dass** als Standard kommerziell erhältliche, über MDA hergestellte DNA verwendet wird.

5. Verfahren oder Verwendung nach mindestens einem der Ansprüche 1-4, **dadurch gekennzeichnet, dass** die Methylierungsanalyse über Restriktionsenzyme erfolgt.

6. Verfahren oder Verwendung nach mindestens einem der Ansprüche 1-5, **dadurch gekennzeichnet, dass** die Methylierungsanalyse nach Umwandlung der DNA in eine Form, in der sich methylierte Cytosine von unmethylierten Cytosinen mittels Hybridisierung unterscheiden lassen, über methylierungsspezifische Ligationsverfahren, MSP, Heavy Methyl oder MethylLight erfolgt.

7. Verfahren oder Verwendung nach mindestens einem der Ansprüche 1-5, **dadurch gekennzeichnet, dass** die Methylierungsanalyse nach Umwandlung der DNA in eine Form, in der sich methylierte Cytosine von unmethylierten Cytosinen mittels Hybridisierung unterscheiden lassen, über Primer-Extension erfolgt.

8. Verfahren oder Verwendung nach mindestens einem der Ansprüche 1-7, **dadurch gekennzeichnet, dass** die Methylierungsanalyse nach Umwandlung der DNA in eine Form, in der sich methylierte Cytosine von unmethylierten Cytosinen mittels Hybridisierung unterscheiden lassen, entweder
a) über eine Amplifikation und eine Hybridisierung der Amplifikate an Oligomer-Mikroarrays erfolgt oder
b) mittels einer Multiplex-PCR erfolgt.

9. Verfahren oder Verwendung nach mindestens einem der Ansprüche 1-8, **dadurch gekennzeichnet, dass** als Standard entweder
a) eine Mischung aus methylierter und nicht-methylierter DNA verwendet wird oder
b) mehrere Gemische aus methylierter und nicht-methylierter DNA mit unterschiedlichen Anteilen an methylierter und nicht-methylierter DNA verwendet werden.

10. Verfahren oder Verwendung nach mindestens einem der Ansprüche 1-9, **dadurch gekennzeichnet, dass** die Methylierungsanalyse entweder
a) zur Diagnose von Krebserkrankungen oder anderen mit einer Veränderung des Methylierungsstatus assoziierten Krankheiten erfolgt oder
b) zur Vorhersage von erwünschten oder unerwünschten Arzneimittelwirkungen und zur Unterscheidung von Zelltypen oder Geweben, oder zur Untersuchung der Zelldifferenzierung erfolgt.

11. Verfahren oder Verwendung nach einem der Ansprüche 1 bis 10, wobei die genomweite Amplifikation unter Einsatz ausschließlich von Nukleotiden bzw. Nukleotidtriphosphaten erfolgt, die nicht-methyliert sind.

12. Ein Kit, der aus bereits über ein WGA-Verfahren amplifizierte DNA sowie aus Reagenzien zur Durchführung einer Bisulfitumwandlung besteht.

13. Kit nach Anspruch 12, wobei die durch ein WGA-Verfahren amplifizierte DNA ausschließlich nicht-methylierte Nukleotide bzw. nicht-methylierte Nukleotidtriphosphate enthält.

14. Kit nach Anspruch 11 oder 12, welches eine Polymerase, Primer und/oder Sonden für eine Amplifikation und Detektion enthält.

## Claims

1. A method for methylation analysis, **characterized in that**
a) a genome-wide amplification is performed,
b) the amplificates generated in a) are used as a standard in the methylation analysis.

2. Use of DNA obtained through a genome-wide amplification method as standard in the methylation analysis.

3. Method or use according to claim 1 or 2, **characterized in that** either
a) PEP,
b) DOP-PCR,
c) linker PCR, or
d) multiple displacement amplification (MDA)
is conducted as amplification method.

4. Method or use according to claim 3, **characterized in that** commercially available DNA is used that was produced via MDA.

5. Method or use according to at least one of claims 1 to 4, **characterized in that** the methylation analysis is performed using restriction enzymes.

6. Method or use according to at least one of claims 1 to 5, **characterized** that the methylation analysis after conversion of the DNA into a form in which methylated cytosine can be distinguished from unmethylated cytosine is using hybridization, is performed via methylation specific ligation methods, MSP, Heavy Methyl or MethylLight.

7. Method or use according to at least one of claims 1 to 5, **characterized** that the methylation analysis after conversion of the DNA into a form in which methylated cytosine can be distinguished from unmethylated cytosine is using hybridization, is performed via primer extension.

8. Method or use according to at least one of claims 1 to 7, **characterized** that the methylation analysis after conversion of the DNA into a form in which methylated cytosine can be distinguished from unmethylated cytosine using hybridization occurs either
a) via an amplification and a hybridization of the amplificates to an oligomer-microarray, or
b) via a multiplex-PCR.

9. Method or use according to at least one of claims 1 to 8, **characterized** that as a standard, either
a) a mixture of methylated and non-methylated DNA is used, or
b) several mixtures from methylated and non-methylated DNA with different fractions of methylated and non-methylated DNA are used.

10. Method or use according to at least one of claims 1 to 9, **characterized** that the methylation analysis is performed either
a) for the diagnosis of cancer diseases or other diseases that are associated with a change of the methylation status, or
b) for the prediction of desired or undesired drug effects and for distinguishing cell types or tissues, or for examining cell differentiation.

11. Method or use according to at least one of claims 1 to 10, wherein the genome-wide amplification is performed using exclusively nucleotides or nucleotide triphosphates that are non-methylated.

12. A kit comprising DNA that was amplified through a WGA method, as well as reagents for performing a bisulfite conversion.

13. The kit according to claim 12, wherein the DNA amplified through a WGA method comprises exclusively non-methylated nucleotides or non-methylated nucleotide triphosphates.

14. The kit according to claim 11 or 12, comprising a polymerase, primer and/or probes for an amplification and detection.

## Revendications

1. Procédé d'analyse de méthylation, **caractérisé en ce que**
a) une amplification à l'échelle du génome est effectuée,
b) les produits d'amplification générés en a) sont utilisés comme standard dans l'analyse de méthylation.

2. Utilisation d'ADN obtenu par des procédés d'amplification à l'échelle du génome comme standard dans l'analyse de méthylation.

3. Procédé ou utilisation selon la revendication 1 ou 2, **caractérisé en ce que** le procédé d'amplification effectué est
a) PEP,
b) DOP-PCR,
c) Linker-PCR, ou
d) Multiple Displacement Amplification (MDA).

4. Procédé ou utilisation selon la revendication 3, **caractérisé en ce qu'**on utilise comme standard de l'ADN obtenu par MDA, disponible dans le commerce.

5. Procédé ou utilisation selon au moins l'une des revendications 1 à 4, **caractérisé en ce que** l'analyse de méthylation est effectuée par l'intermédiaire d'enzymes de restriction.

6. Procédé ou utilisation selon au moins l'une des revendications 1 à 5, **caractérisé en ce que** l'analyse de méthylation est effectuée, après transformation de l'ADN en une forme dans laquelle des cytosines méthylées se distinguent de cytosines non méthylées au moyen d'hybridation, par l'intermédiaire de procédés de ligation spécifiques à la méthylation, MSP, Heavy Methyl ou MethylLight.

7. Procédé ou utilisation selon au moins l'une des revendications 1 à 5, **caractérisé en ce que** l'analyse de méthylation est effectuée, après transformation de l'ADN en une forme dans laquelle des cytosines méthylées se distinguent de cytosines non méthylées au moyen d'hybridation, par extension d'amorce.

8. Procédé ou utilisation selon au moins l'une des revendications 1 à 7, **caractérisé en ce que** l'analyse de méthylation est effectuée, après transformation de l'ADN en une forme dans laquelle des cytosines méthylées se distinguent de cytosines non méthylées au moyen d'hybridation,
a) soit par une amplification et une hybridation des produits d'amplification sur des microréseaux d'oligomères,
b) soit au moyen d'une PCR multiplex.

9. Procédé ou utilisation selon au moins l'une des revendications 1 à 8, **caractérisé en ce que** sont utilisés, comme standard,
a) soit un mélange d'ADN méthylé et non méthylé,
b) soit plusieurs mélanges d'ADN méthylé et non méthylé avec des fractions différentes d'ADN méthylé et non méthylé.

10. Procédé ou utilisation selon au moins l'une des revendications 1 à 9, **caractérisé en ce que** l'analyse de méthylation est effectuée
a) soit pour le diagnostic de maladies cancéreuses ou d'autres maladies associées à une modification de l'état de méthylation,
b) soit pour la prédiction d'effets souhaités ou non souhaités de médicaments et pour distinguer des types de cellules ou des tissus, soit pour examiner la différenciation des cellules.

11. Procédé ou utilisation selon l'une des revendications 1 à 10, l'amplification à l'échelle du génome étant effectuée en utilisant exclusivement des nucléotides ou triphosphates nucléotides qui ne sont pas méthylés.

12. Kit qui est constitué d'ADN déjà amplifié par un procédé WGA, ainsi que de réactifs pour effectuer une transformation de bisulfite.

13. Kit selon la revendication 12, dans lequel l'ADN amplifié par un procédé WGA contient exclusivement des nucléotides non méthylés ou des triphosphates nucléotides non méthylés.

14. Kit selon la revendication 11 ou 12, qui contient une polymérase, une amorce et/ou des sondes pour une amplification et une détection.
